# FASCICULE DE BREVET EUROPEEN

(11) **EP 0 632 714 B1**
(45) Date de publication et mention de la délivrance du brevet: **13.08.1997**
(21) Numéro de dépôt: 94905151.0
(22) Date de dépôt: 25.01.1994
(51) Int. Cl.: A61K 7/043

(54) **VERNIS A ONGLES, INCOLORE OU COLORE, A SECHAGE RAPIDE**
SCHNELLTROCKNENDER FARBIGER ODER FARBLOSER NAGELLACK
QUICK-DRYING COLOURED OR COLOURLESS NAIL VARNISH

(30) Priorité: 25.01.1993 FR 9300692
(43) Date de publication de la demande: 11.01.1995
(73) Titulaire: L'OREAL, 75008 Paris (FR)
(72) Inventeur: DE LA POTERIE, Valérie, F - 77820 Le Chatelet en Brie (FR); MELLUL, Myriam, F-94240 L'Hay-les-Roses (FR)
(74) Mandataire: Stalla-Bourdillon, Bernard
(86) Numéro de dépôt international: FR9400086
(87) Numéro de publication internationale: WO9416670

(56) Documents cités:
- EP-A- 0 295 780
- EP-A- 0 432 572
- EP-A- 0 558 423
- FR-A- 2 379 280
- FR-A- 2 397 186
- FR-A- 2 679 445
- GB-A- 1 199 776
- US-A- 5 045 309
- PATENT ABSTRACTS OF JAPAN vol. 12, no. 462 (C-549) (3309) 5 Décembre 1988 & JP,A,63 183 508 (NIPPON OIL & FATS CO LTD) 28 Juillet 1988

## Description

La présente invention a pour objet un vernis à ongles, incolore ou coloré, contenant en plus des ingrédients usuels l'association d'une amino- ou thio- silicone et d'un composé organofluoré hydrocarboné.

Parmi les principales caractéristiques que doivent posséder les vernis à ongles, on doit tout particulièrement mentionner l'absence d'irritation de la peau et des ongles, l'obtention d'un film homogène et d'un bon brillant mais également une bonne adhérence sur la surface de l'ongle ainsi qu'une certaine flexibilité et résistance du film, ceci en vue d'éviter sa fragilité pouvant conduire à une craquelure du vernis.

De façon générale, on utilise à l'heure actuelle pour conférer une bonne adhérence, des résines modifiées et des plastifiants, ce qui conduit à une bonne flexibilité du vernis.

En vue d'améliorer l'adhérence, il a également été proposé l'application d'une précouche d'accrochage ou encore l'introduction de certains additifs permettant d'améliorer l'adhérence.

L'utilisation de silicone dans les vernis à ongles en tant qu'additif a déjà été décrite dans le but d'améliorer la résistance à l'eau et de faciliter l'étalement du vernis.

Ainsi, dans US 4.873.077, il a été proposé l'utilisation d'une silicone fluide en vue d'améliorer la douceur du film après évaporation du solvant et d'améliorer sa résistance à l'humidité.

De même, dans JP-A-55.193316 et 59.199621, il a également été proposé l'utilisation de silicones, en particulier de méthyle ou diméthylpolysiloxane pour améliorer le brillant et la résistance à l'eau et à l'abrasion du film de vernis.

Dans FR-78.02721 (2.379.280) ont été décrites des compositions de vernis à ongles formant des films facilement détachables des ongles sans l'aide de produit dissolvant.

Selon ce brevet, la composition de vernis contient, en plus des ingrédients usuels, de 0,01 à 30 parties en poids, par rapport à la résine naturelle ou synthétique, d'un organopolysiloxane porteur de diverses fonctions dont éventuellement des fonctions amines. La présence de l'organopolysiloxane est essentielle en vue d'obtenir un film facilement détachable ou pelable de la surface de l'ongle.

L'utilisation de composés organofluorés dans des vernis à ongles et plus particulièrement de résines fluorocarbonées n'a été préconisée dans EP 370.470 que comme liant de pigments fluorescents.

US-A-5 045 309 décrit un vernis à ongles à séchage rapide contenant 25-50% en poids d'un composé organofluoré hydrocarboné. Après d'importantes recherches, on a maintenant trouvé de façon tout à fait surprenante et inattendue, qu'en utilisant dans une composition de vernis à ongles une certaine classe bien déterminée d'organopolysiloxanes à fonction(s) amine(s) ou thiol(s) ci-après désignés par les expressions "amino-silicones" et "thio-silicones" en association avec un composé organofluoré hydrocarboné, il était possible d'améliorer de façon particulièrement significative, le temps de séchage des vernis, incolores ou colorés, sur les ongles ainsi que, le plus souvent, leur adhérence.

La présente invention a donc pour objet à titre de produit industriel nouveau, un vernis à ongles, incolore ou coloré, contenant dans un système solvant pour vernis, une substance filmogène, une résine et un agent plastifiant, ledit vernis contenant en outre, en vue d'améliorer le temps de séchage, l'association de (i) 0,05 à 5% en poids d'une amino- ou thio-silicone et de (ii) 0,05 à 5% en poids d'un composé organofluoré hydrocarboné, le poids total de (i) + (ii) devant être inférieur ou égal à 5% en poids du poids total du vernis. Les amino-silicones du vernis selon l'invention peuvent être représentées par la formule générale suivante : 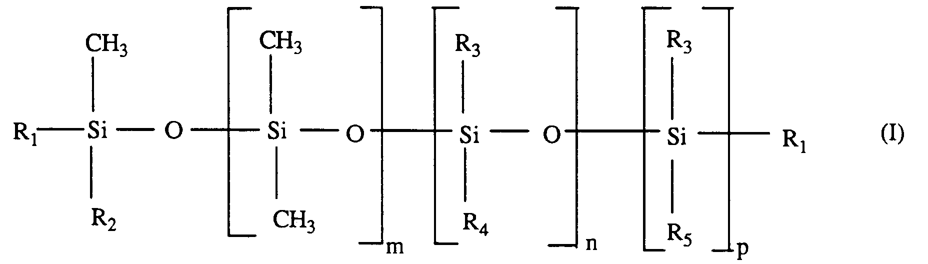 dans laquelle :
R₁ représente CH₃, OCH₃, OCH₂CH₃ ou ―(CH₂)̵_{d}―NH₂
R₂ représente CH₃, OCH₃ ou OCH₂CH₃,
R₃ représente CH₃, OCH₃, OCH₂CH₃ ou OSi(CH₃)₃,
R₄ représente CH₃ ou 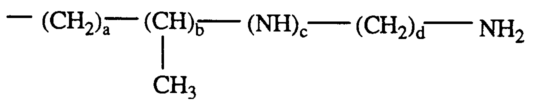
R₅ représente CH₃, OCH₃, OCH₂CH₃ ou 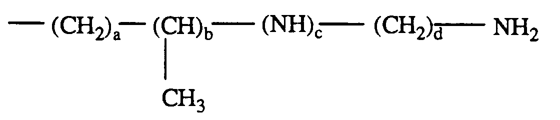
   a est 0 à 6
   b est 0 à 2
   c est 0 ou 1
   d est 1 à 6
sous réserve que :
(i) lorsque R₁ représente le radical -(CH₂)_{d}-NH₂, R₄ représente CH₃ et R₅ représente CH₃, OCH₃, ou OCH₂CH₃,
(ii) lorsque R₄ représente CH₃, R₅ représente : 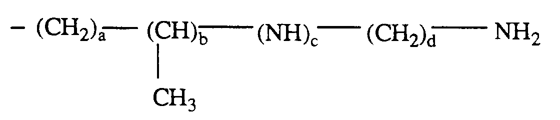 et
(iii) lorsque R₄ représente 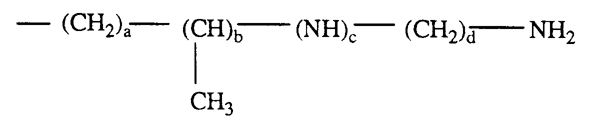
   R₅ représente CH₃, OCH₃ ou OCH₂CH₃,
   m est 1 à 1000
   n est 0 à 50 et
   p est 0 à 50
   n et p ne pouvant simultanément être 0,
le poids moléculaire de la dite amino-silicone étant compris entre 500 et 100.000.

De préférence, l'amino-silicone doit présenter un indice d'amine (meq/g) compris entre 0,05 et 2,3 et de préférence 0,05 et 0,50.

Le poids moléculaire de l'amino-silicone de Formule (I) est de préférence compris entre 1000 et 20.000.

Parmi les amino-silicones de Formule (I), on peut notamment citer celles vendues par la Société Shin-Etsu sous les dénominations "KF860", "KF861", "KF862", "KF864", "KF865", "KF867", "X22-3680" et "X22-3801C", celles vendues par la Société Wacker sous les dénomination "SLM 55051", "L656", "SLM 55067", "VP1653" et "VP1480M" celle vendue par la Société Dow Corning sous la dénomination de "DC929", celles vendues par la Société Goldschmidt sous les dénominations de "TEGOMER A-Si2320" ou "TEGOMER A-Si2120", et celle vendue par la Société General Electric sous la dénomination de "SF 1706".

Les amino-silicones peuvent se présenter sous forme d'huile pure ou sous forme d'émulsion aqueuse comportant de 5 à 50% de matière active telles que le produit "QS-7224" vendu par la Société Dow Corning ou le produit "SLM 23047" vendu par la Société Wacker.

Les thio-silicones des vernis selon l'invention sont des polydiorganosiloxanes comportant dans leur molécule au moins une unité de formule : 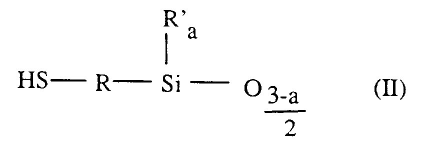 dans laquelle :
R désigne un radical aliphatique divalent ayant de 2 à 26 atomes de carbone, éventuellement interrompu par une fonction ester et pouvant porter des motifs d'oxyde d'éthylène ou d'oxyde de propylène ou leur mélange,
R' désigne un radical monovalent hydrocarbonné ayant 1 à 6 atomes de carbone, alcoxy ayant 1 à 4 atomes de carbone ou triméthylsilyloxy,
a est égal à 0, 1 ou 2,
les unités restantes ayant pour formule :
1) 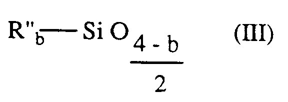 dans laquelle :
   R'' représente un groupe alkyle en C₁-C₁₈, phénylalkyle en C₁-C₆ ou phényle,
   b est égal à 1, 2 ou 3,
   au moins 50% des groupements R' et R'' représentant un groupement méthyle et
2) éventuellement des unités de formule : 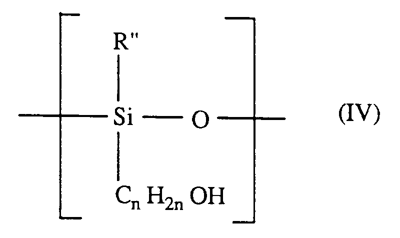 dans laquelle
   R'' est tel que défini ci-dessus et n est un nombre entier de 1 à 18.

Le taux pondéral des groupements thiols présents dans le polydiorganosiloxane est compris de préférence entre 0,1 et 15% et en particulier entre 0,15 et 13%.

Selon une forme de réalisation particulière le radical R représente un groupement -(CH₂)ₙ- où n est compris entre 3 et 8, ou un groupement de formule :

―CₙH₂ₙO(CₓH₂ₓO)ₚ―COCₘH₂ₘ―

dans laquelle :
n représente un nombre entier compris entre 1 et 18, m représente un nombre entier compris entre 1 et 8, x = 2 ou 3 et lorsque x = 3, le radical C₃H₆ est ramifié et p est égal à 0 ou désigne un nombre pouvant aller jusqu'à 40, et
R' représente un radical alkyle en C₁-C₆ tel que méthyle, éthyle, n-propyle, isopropyle, n-butyle.

De préférence, le radical R'' représente un groupement méthyle et le radical R représente -(CH₂)₂-, -(CH₂)₃- ou -(CH₂)₄-.

Le nombre total des unités de formule (II), (III) et éventuellement (IV) est de préférence égal ou inférieur à 500 et en particulier compris entre 10 et 500.

De telles thio-silicones sont notamment décrits dans GB-1.182.939, GB-1.199.776, FR-1.356.552, 1.378.791, et EP-A-295780 et FR-A-2.611.730.

Parmi les thio-silicones, telles que définies ci-dessus on peut citer celles vendues par la Société Wacker sous les dénominations de "VP1641" ou "SLM 50253/6", celles vendues par la Société Shin-Etsu sous les dénominations de "X22-980" et "X22-167B" et celles vendues par la Société Genesee sous les dénominations de "GP72-A" et "GP71".

Le composé organofluoré hydrocarboné du vernis selon l'invention est non volatil, son point d'ébullition étant supérieur à 30°C.

De tels composés organofluorés hydrocarbonés ont une structure chimique comportant un squelette carboné où certains atomes d'hydrogène ont été substitués par des atomes de fluor, le squelette carboné pouvant comporter un ou plusieurs hétéroatomes et un ou plusieurs groupements organiques fonctionnels.

Pour les composés organofluorés hydrocarbonés, on définit le taux de substitution des atomes d'hydrogène par des atomes de fluor, sous la forme du rapport : nombre d'atomes de fluor/(nombre d'atomes de fluor+nombre d'atomes d'hydrogène) où seuls les atomes d'hydrogène liés aux atomes de carbone du squelette sont pris en compte.

Les composés organofluorés hydrocarbonés tels que définis ci-dessus peuvent être représentés par la formule générale suivante :

(R_{F})ₓ-(A)_{y}-(R_{H})_{z} (V)

dans laquelle :
x représente 1, 2 ou 3,
y représente 0 ou 1,
z représente 0, 1, 2 ou 3,
à la condition que y et z ne soient pas simultanément 0, et que lorsque z est 0, x est 2 ou 3,
R_{F} représente un radical fluoré aliphatique ou aromatique, saturé ou insaturé, à chaîne linéaire, ramifiée ou cyclique, cette chaîne pouvant être fonctionnalisée et/ou être interrompue par des atomes divalents tels que l'oxygène ou le soufre, ou trivalents tels que l'azote et/ou substituée par des atomes d'hydrogène ou d'autres atomes d'halogène, à la condition que, pour deux atomes de carbone du squelette, ne soit pas présent plus d'un de ces substituants autres que le fluor,
R_{H} représente un radical hydrocarboné aliphatique ou aromatique, saturé ou insaturé, à chaîne linéaire, ramifiée ou cyclique, cette chaîne pouvant être fonctionnalisée et/ou interrompue par un ou plusieurs atomes divalents tels que l'oxygène ou le soufre ou par un ou plusieurs atomes trivalents comme l'azote,
A représente un radical di, tri ou tétravalent tel que 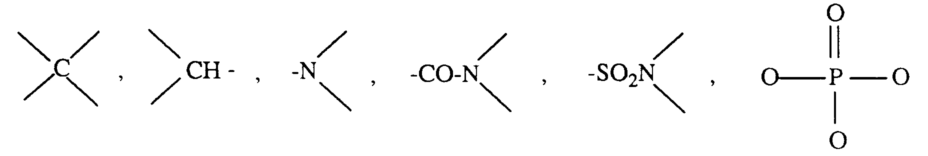 les structures cycliques, aliphatiques ou aromatiques ou les insaturations éthyléniques.

Par l'expression "fonctionnalisé", on entend selon l'invention, une substitution du squelette intercalaire, terminale ou pendante, par au moins un groupement organique fonctionnel comme une fonction alcool, thiol, acide, carbonyle, sulfoxyde, ester, amide, amine, phosphate, éthylénique, acétylénique, et énamine ou sulfonamide.

Par insaturation éthylénique, on entend par exemple

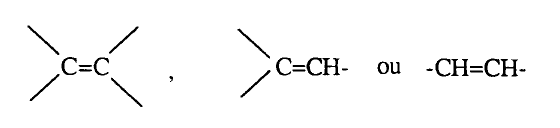

De préférence, R_{H} représente un radical alkyle linéaire ou ramifié en C₁-C₂₂ ou un mélange de radicaux alkyles linéaires ou ramifiés en C₁-C₂₂, un radical aryle en C₆-C₁₀ ou un radical aralkyle en C₇-C₁₅.

De préférence, R_{F} représente un radical perfluoroalkyle ayant de 4 à 22 atomes de carbone.

Selon l'invention, les composés organofluorés hydrocarbonés utilisés ont de préférence un taux de substitution compris entre 0,5 et 95 % mais de préférence supérieur à 10% et inférieur à 80%.

A titre illustratif, on peut citer les composés possédant des groupes perfluorocarbures et des groupes hydrocarbures, le nombre total de carbone étant compris entre 10 et 30, le nombre d'atomes de carbone des groupes hydrocarbures étant étal ou supérieur à deux fois le nombre d'atomes de carbone des groupes perfluorocarbures, de tels composés étant décrits dans JP-A-63.002916.

De même, à titre illustratif, on peut citer les composés organofluorés hydrocarbonés dont la structure générale est définie par la formule (VI) suivante :

R₁-(CH₂)ₙ-X-[C₃H₅(OH)]-(Y)ₓ―(CH₂)ₘ―R₂ (VI)

dans laquelle :
le radical divalent C₃H₅(OH) représente l'un au moins des radicaux divalents suivants : 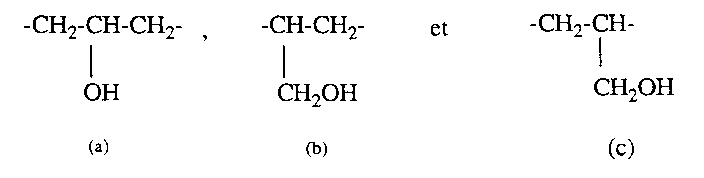
R₁ représente un radical alkyle perfluoré, linéaire ou ramifié, en C₄-C₂₀ ou un mélange de tels radicaux alkyles perfluorés, linéaires ou ramifiés, en C₄-C₂₀,
R₂ représente R₁ ou un radical alkyle, linéaire ou ramifié, en C₁-C₂₂ ou un mélange de tels radicaux alkyles, linéaires ou ramifiés, en C₁-C₂₂ ou un radical aryle en C₆-C₁₀ ou aralkyle en C₇-C₁₅, sous réserve que :
   (i) lorsque R₂ est différent de R₁,
      X et Y représentent -O-, -S-, 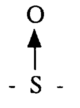 ou 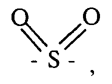 X et Y ne pouvant représenter simultanément 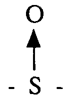 ou 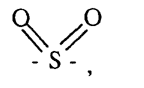 et
   (ii) lorsque R₂ représente R₁, X et Y, identiques, représentent -O- ou -S-
      m et n, identiques ou différents, sont compris entre 0 et 4 et
      x représente O ou 1.

Les composés de formule (VI) ci-dessus sont plus particulièrement décrits dans FR 91 15019, EP-A-166.696, DE 2.702.607, JP 89-193.236, JP 92-275.268 et US 3.893.984.

Comme exemples de composés de formule (VI), on peut citer :
le 1-(2'-F-hexyl-éthylthio)-3-(2''-éthylhexyloxy)-2-propanol,
le 1-(2'-F-octyl-éthylthio)-3-(2''-éthylhexyloxy)-2-propanol,
le 1-(2'-F-octyl-éthylthio)-3-butyloxy-2-propanol,
le 1-(2'-F-octyl-éthylthio)-3-phénoxy-2-propanol,
le 1-(2'-F-hexyl-éthylthio)-3-dodécyloxy-2-propanol,
le 1-(2'-F-hexyl-éthylthio)-2-décanol,
le 1-(2'-F-hexyl-éthylthio)-2'-hexanol,
le 1-(2'-F-octyl-éthylthio)-2-hexanol,
le 1-(2'-F-hexyl-éthyloxy)-3-(2''-éthylhexyloxy)-2-propanol,
le 1-(2'-F-hexyl-éthylthio)-3-octylthio-2-propanol,
le 1-(2'-F-octyl-éthylthio)-3-octadécylthio-2-propanol,
le 1,3-bis-(2'-F-hexyl-éthylthio)-2-propanol, et
le 1,3-bis-(2'-F-octyl-éthylthio)-2-propanol.

Comme autres composés organofluorés hydrocarbonés on peut également citer ceux répondant au formules (X) et (XI) suivantes : 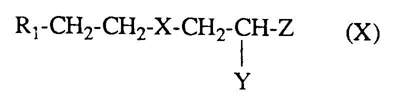 dans laquelle :
R₁ représente un radical alkyle perfluoré, linéaire ou ramifié, en C₄-C₂₀ ou un mélange de tels radicaux alkyles perfluorés, linéaires ou ramifiés, en C₄-C₂₀.
Y est
   soit (i) OH et Z représente -CH₃, -CH₂OH, -CH₂OCOCH₃ ou -C₆H₅,
   soit (ii) -CH₂OH et Z représente -OCOCH₃, et
X représente -O-, -S-, 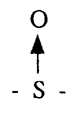 ou

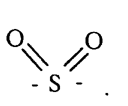

   R₁-CH=CH-CH₂-O-[C₃H₅(OH)]―W (XI)
dans laquelle :
le radical divalent C₃H₅(OH) représente l'un au moins des radicaux divalents suivants : 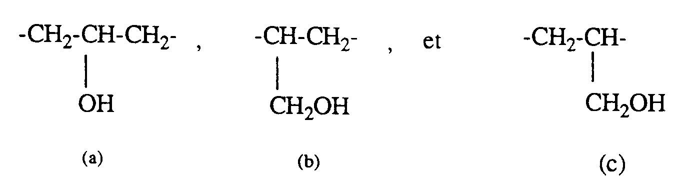
R₁ représente un radical alkyle perfluoré, linéaire ou ramifié, en C₄-C₂₀ ou un mélange de tels radicaux alkyles perfluorés linéaires ou ramifiés, en C₄-C₂₀,
W représente -OR, -SR, -COOR, -O-C₆H₅, o- ou p-O-C₆H₄(CH₃) ou o- ou p-O-C₆H₄(OH), R représentant un radical alkyle, linéaire ou ramifié, en C₁-C₁₈.

Les composés de formule (X) sont plus particulièrement décrits dans DE 2.052.579 et ceux de formule (XI) dans US 3.952.066.

Parmi ceux-ci on peut notamment citer :
le 2-(2'-F-hexyl-éthylthio)-1-phényl-éthanol,
le 2-(2'-F-hexyl-éthylsulfonyl)-1-phényl-éthanol,
le 1-(3'-F-hexyl-2'-propénoxy)-3-octylthio-2-propanol,
le 1-(3'-F-hexyl-2'-propénoxy)-3-butyloxy-2-propanol, et
le 1-(3'-F-octyl-2'-propénoxy)-3-octadécylthio-2-propanol

En outre on peut utiliser, selon l'invention, comme composés organofluorés hydrocarbonés, ceux vendus sous la dénomination de "Nofable Fo" par la Société Nippon Oil & Co., ayant la formule suivante : 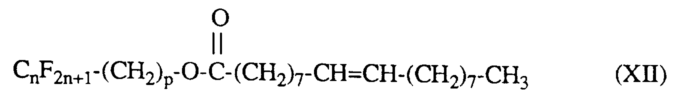 dans laquelle :
n est un nombre entier égal à 6 ou 8 et p est 1 ou 2.

Enfin on peut également citer les composés organofluorés répondant à la formule (XIII) suivante :

R-CH=CH-CH₂-CₙF₂ₙ₊₁ (XIII)

dans laquelle :
R désigne un radical butyle ou phényle, et
n est 4, 6 ou 8.

De tels composés sont décrits dans B. Escoula, Synthetic Communications, 15(1), 35-38 (1985).

Les composés organofluorés hydrocarbonés utilisés selon l'invention se présentent de préférence sous forme d'huile ou de cire.

L'association de l'amino- ou thio-silicone et du composé organofluoré hydrocarboné permet, comme le montrent les exemples donnés ci-après, de réduire d'au moins 50% environ le temps de séchage et peut provoquer une forte augmentation de l'adhérence du vernis sur les ongles.

Selon l'invention, il est également possible d'associer au vernis, une gomme de silicone ou une résine de silicone en un faible pourcentage de l'ordre de 0,1 à 2 % en poids et de préférence de 0,3 % en poids pour améliorer l'étalement, l'aspect lisse du film et la brillance.

Selon l'invention, le système solvant du vernis est généralement présent en une proportion comprise entre 55 et 90 % en poids par rapport au poids total du vernis.

Ce système solvant est essentiellement constitué par un mélange de divers solvants organiques volatils, ceci en vue d'obtenir des temps de séchage relativement courts.

Parmi ces solvants on peut citer l'acétone, l'acétate d'éthyle, l'acétate de butyle, l'acétate de méthoxy-2 éthyle, la méthyl-éthyl-cétone, la méthyl-isobutyl-cétone, l'acétate de méthyle, l'acétate d'amyle et l'acétate d'isopropyle.

Le système solvant peut comprendre également un diluant qui est de préférence un hydrocarbure linéaire ou ramifié saturé, tel que l'hexane ou l'octane ou encore un hydrocarbure aromatique tel que le toluène ou le xylène dans une proportion généralement comprise entre 10 et 35 % par rapport au poids total du vernis.

Le système solvant peut également inclure d'autres solvants volatils tels que de l'éthanol, du n-butanol, du n-propanol, de l'isopropanol ou leurs mélanges.

La substance filmogène est généralement présente dans le vernis selon l'invention à une concentration comprise entre 5 et 20 % et de préférence entre 10 et 20 % en poids par rapport au poids total du vernis.

Parmi les substances filmogènes préférées, on peut citer les nitrocelluloses du type "RS" ou "SS" et en particulier la nitrocellulose type 1/4 seconde RS, la nitrocellulose type 1/2 seconde RS, la nitrocellulose type 1/2 seconde SS et la nitrocellulose type 3/4 seconde RS.

Comme substance filmogène secondaire, on peut également utiliser selon l'invention des dérivés polyvinyliques tels que le butyrate de polyvinyle, ainsi que les copolymères décrits dans FR-80.07328, 81.03199 et 88.08172.

Selon l'invention, l'agent plastifiant est généralement présent dans le vernis à une concentration comprise entre 2 et 10 % en poids par rapport au poids total du vernis.

Les agents plastifiants permettent de régler la flexibilité du film sans affaiblir sa résistance ou sa force physique. Parmi les agents plastifiants susceptibles d'être utilisés dans les vernis selon l'invention on peut citer : le phosphate de tricrésyle, le benzoate de benzyle, le phosphate de tributyle, l'acétyl-ricinoléate de butyle, l'acétyl-ricinoléate de glycéryle, le phtalate de dibutyle, le glycolate de butyle, le phtalate de dioctyle, le stéarate de butyle, le phosphate de tributoxy-éthyle, le phosphate de triphényle, le citrate de triéthyle, le citrate de tributyle, l'acétyl-citrate de tributyle, l'acétyl-citrate de triéthyl-2 hexyle, le tartrate de dibutyle, le phtalate de diméthoxyéthyle, le phtalate de di-isobutyle, le phtalate de diamyle, le camphre, le triacétate de glycérol et leurs mélanges.

Selon l'invention, le vernis à ongles contient également une résine généralement présente en une proportion comprise entre 0,5 et 15 % en poids par rapport au poids total du vernis.

Parmi les nombreuses résines utilisables, on préfère employer selon l'invention des résines du type aryl-sulfonamide formaldéhyde ou aryl-sulfonamide époxy, notamment la résine toluène sulfonamide formaldéhyde plus connue sous les dénominations commerciales de "Santolite MHP", "Santolite MS 80 %" et "Ketjenflex MS80" ou encore des résines alkydes telles que celles vendues par la Société Dainippon sous la dénomination de "Beckosol ODE 230-70".

Ces résines, tout en augmentant le pouvoir filmogène, améliorent le brillant ainsi que l'adhérence.

Lorsque le vernis à ongles selon l'invention est un vernis coloré, on utilise alors au moins un pigment de nature organique ou inorganique.

Parmi les pigments organiques, on peut citer les D & C Red n°10, 11, 12 et 13, le D & C Red n°7, les D & C Red n°5 et 6, le D & C Red n°34, des laques telles que la laque D & C Yellow n°5 et la laque D & C Red n°2.

Parmi les pigments inorganiques, on peut citer le dioxyde de titane, l'oxychlorure de bismuth, l'oxyde de fer brun et les oxydes de fer rouge. On peut citer comme pigment organique la guanine.

Selon cette forme de réalisation, les pigments sont généralement présents en une proportion comprise entre 0,01 et 2 % en poids par rapport au poids total du vernis.

Enfin, en vue d'éviter la sédimentation des pigments, certains agents thixotropes peuvent être employés, tels que par exemple la "Bentone 27" ou la "Bentone 38".

Les vernis à ongles selon l'invention peuvent en outre contenir des adjuvants couramment utilisés dans les vernis à ongles. Parmi ces adjuvants, on peut citer les filtres U.V. tels que les dérivés de benzophénone et le 2-cyano-3,3-diphényl acrylate d'éthyle.

On va maintenant donner à titre d'illustration plusieurs exemples de vernis à ongles selon l'invention.

### EXEMPLES DE PREPARATION

### EXEMPLE 1 : 1-(2'-F-hexyl-éthylthio)-3-(2''-éthylhexyloxy)-2-propanol

A une température de 25°C, sous agitation et sous courant d'azote, on ajoute en une minute 3,6 g d'une solution méthanolique de méthylate de sodium (environ 30 % - 5,54 meq g⁻¹) à 152 g de 2-F-hexyl-éthanethiol.

Le mélange est chauffé à 70°C puis on évapore sous vide le méthanol présent dans le milieu.

Le 2-éthylhexylglycidyléther (74,4 g) est ensuite ajouté goutte à goutte en une heure. On maintient la température du mélange entre 60 et 70°C durant l'addition de l'époxyde.

A la fin de l'addition, la température est amenée à 25°C et le mélange est neutralisé à l'aide de 20 ml de HCl Normal et le 1-(2'-F-hexyl-éthylthio)3-(2''-éthylhexyloxy)2-propanol est séparé par distillation : Eb = 141°C/66,5 Pa.

On obtient 175 g (77 %) d'une huile translucide incolore.

| Analyse élémentaire : | | | | |
|---|---|---|---|---|
| | C% | H% | S% | F% |
| Calculé | 40,28 | 4,80 | 5,66 | 43,60 |
| Trouvé | 40,37 | 4,82 | 5,55 | 43,74 |

### EXEMPLE 2 : 1-(2'-F-octyl-éthylthio)-3-phénoxy-2-propanol

A une température de 25°C, sous agitation et sous courant d'azote, on additionne 2,65g d'une solution méthanolique de méthylate de sodium (5,65 meq g⁻¹) à 144g (0,3 mole) de 2-F-octyl-éthanethiol en solution dans 250ml d'éther diisopropylique.

On chauffe le mélange en maintenant la température entre 55 et 65°C et on ajoute goutte à goutte, en 45 minutes, 45g (0,3 mole) de phénylglycidyléther.

En fin de réaction, le mélange est neutralisé par 15ml de HCl normal.

Après évaporation du solvant, on obtient un produit pâteux de couleur jaune pâle.

Le 1-(2'-F-octyl-éthylthio)3-phénoxy-2-propanol est purifié par distillation (166-168°C/13,3 Pa).

On obtient après refroidissement et cristallisation 115g d'un solide blanc.
Rendement : 65%
Point de fusion : 64°C

| Analyse élémentaire : | | | | |
|---|---|---|---|---|
| | C% | H% | S% | F% |
| Calculé | 36,20 | 2,40 | 5,09 | 51,24 |
| Trouvé | 36,20 | 2,38 | 4,94 | 51,19 |

### EXEMPLES DE VERNIS A ONGLES

| **EXEMPLE 1** | |
|---|---|
| Nitrocellulose | 10,84 % |
| Résine toluène sulfonamide formaldéhyde | 9,76 % |
| Acétylcitrate de tributyle | 6,51 % |
| Toluène | 30,97 % |
| Acétate de butyle | 21,685 % |
| Acétate d'éthyle | 9,29 % |
| Alcool isopropylique | 7,74 % |
| Stéaralkonium hectorite | 1,35 % |
| Pigments | 1,00 % |
| Acide citrique | 0,055 % |
| 1-(2'-F-hexyl-éthylthio)-3-(2''-éthylhexyloxy)-2-propanol | 0,5 % |
| Amino-silicone "KF 865" de la Société Shin-Etsu | 0,3 % |
| Temps de séchage (30°C-50 % humidité relative) = 15 minutes | |

| **EXEMPLE 2 : (comparatif)** | |
|---|---|
| Nitrocellulose | 10,93 % |
| Résine toluène sulfonamide formaldéhyde | 9,85 % |
| Acétylcitrate de tributyle | 6,565 % |
| Toluene | 31,22 % |
| Acétate de butyle | 21,86 % |
| Acétate d'éthyle | 9,36 % |
| Alcool isopropylique | 7,80 % |
| Stéaralkonium hectorite | 1,36 % |
| Pigments | 1,00 % |
| Acide citrique | 0,055 % |
| Temps de séchage (30°C-50 % humidité relative) = 27 minutes | |

Le temps de séchage de ce vernis ne contenant pas l'association d'une amino-silicone et d'un composé organofluoré hydrocarboné est beaucoup plus long que celui du vernis de l'Exemple 1.

| **EXEMPLE 3 (comparatif)** | |
|---|---|
| Nitrocellulose | 10,84 % |
| Résine toluène sulfonamide formaldéhyde | 9,76 % |
| Acétylcitrate de tributyle | 6,51 % |
| Toluène | 30,97 % |
| Acétate de butyle | 21,685 % |
| Acétate d'éthyle | 9,29 % |
| Alcool isopropylique | 7,74 % |
| Stéaralkonium hectorite | 1,35 % |
| Pigments | 1,00 % |
| Acide citrique | 0,055 % |
| 1-(2'-F-hexyl-éthylthio)-3-(2''-éthylhexyloxy)-2-propanol | 0,5 % |
| Phényldiméthicone "BELSIL PDM 200" de la Société Wacker | 0,3 % |
| Temps de séchage (30°C-50% d'humidité relative) = 22 minutes | |

Cet exemple comparatif montre que la présence d'une silicone du type non amino fonctionnelle (phényldiméthicone) ne permet pas de réduire le temps de séchage.

| **EXEMPLE 4 (comparatif)** | |
|---|---|
| Nitrocellulose | 10,9 % |
| Résine toluène sulfonamide formaldéhyde | 9,8 % |
| Acétylcitrate de tributyle | 6,495 % |
| Toluène | 31,105 % |
| Acétate de butyle | 21,8 % |
| Acétate d'éthyle | 9,395 % |
| Alcool isopropylique | 7,8 % |
| Stéaralkonium hectorite | 1,35 % |
| Pigments | 1,00 % |
| Acide citrique | 0,055 % |
| Amino-silicone "KF 865" de la Société Shin-Etsu | 0,3 % |
| Temps de séchage (30°C-50 % humidité relative) = 18 minutes | |

Cet exemple ne contenant pas de composé organofluoré hydrocarboné permet de montrer, par rapport à l'exemple 1, que le temps de séchage est augmenté de plus de 15%.

| **EXEMPLE 5 (comparatif)** | |
|---|---|
| Nitrocellulose | 10,88 % |
| Résine toluène sulfonamide formaldéhyde | 9,779 % |
| Acétylcitrate de tributyle | 6,481 % |
| Toluène | 31,04 % |
| Acétate de butyle | 21,76 % |
| Acétate d'éthyle | 9,375 % |
| Alcool isopropylique | 7,78 % |
| Stéaralkonium hectorite | 1,35 % |
| Pigments | 1,00 % |
| Acide citrique | 0,055 % |
| 1-(2'-F-hexyl-éthylthio)-3-(2''-éthylhexyloxy)-2-propanol | 0,5 % |
| Temps de séchage (30°C-50 % humidité relative) = **25** minutes | |

Cet exemple montre que le composé organofluoré hydrocarboné seul n'a pratiquement aucune influence sur la réduction du temps de séchage par rapport à l'exemple 2 (comparatif).

| **EXEMPLE 6** | |
|---|---|
| Nitrocellulose | 10,76 % |
| Résine toluène sulfonamide formaldéhyde | 9,69 % |
| Acétylcitrate de tributyle | 6,455 % |
| Toluène | 30,75 % |
| Acétate de butyle | 21,53 % |
| Acétate d'éthyle | 9,23 % |
| Alcool isopropylique | 7,68 % |
| Stéaralkonium hectorite | 1,35 % |
| Pigments | 1,00 % |
| Acide citrique | 0,055 % |
| 1-(2'-F-hexyl-éthylthio)-3-phénoxy-2-propanol | 1,0 % |
| Amino-silicone "KF 865" de la Société Shin-Etsu | 0,5 % |
| Temps de séchage (30°C-50 % humidité relative) = 12 minutes | |

| **EXEMPLE 7** | |
|---|---|
| Nitrocellulose | 10,84 % |
| Résine toluène sulfonamide formaldéhyde | 9,76 % |
| Acétylcitrate de tributyle | 6,51 % |
| Toluène | 30,97 % |
| Acétate de butyle | 21,685 % |
| Acétate d'éthyle | 9,29 % |
| Alcool isopropylique | 7,74 % |
| Stéaralkonium hectorite | 1,35 % |
| Pigments | 1,00 % |
| Acide citrique | 0,055 % |
| Amino-silicone "KF 865" de la Société Shin-Etsu | 0,5 % |
| Composé organo fluoré hydrocarboné "NOFABLE FO 9982" de la Société Nippon Oils | 0,3 % |
| Temps de séchage (30°C-50 % humidité relative) = 14,5 minutes | |

| **EXEMPLE 8** | |
|---|---|
| Nitrocellulose | 10,84 % |
| Résine toluène sulfonamide formaldéhyde | 9,76 % |
| Acétylcitrate de tributyle | 6,51 % |
| Toluène | 30,97 % |
| Acétate de butyle | 21,685 % |
| Acétate d'éthyle | 9,29 % |
| Alcool isopropylique | 7,74 % |
| Stéaralkonium hectorite | 1,35 % |
| Pigments | 1,00 % |
| Acide citrique | 0,055 % |
| Amino-silicone "KF 865" de la Société Shin-Etsu | 0,5 % |
| 1-(2'-F-hexyl-éthylthio)-3-(2''-éthylhexyloxy)-2-propanol | 0,3 % |
| Temps de séchage (30°C-50 % humidité relative) = 13 minutes | |

Un test effectué sur un panel de 60 femmes avec ce vernis a montré que les utilisatrices avaient constaté une très nette amélioration du temps de séchage par rapport au même vernis mais ne contenant pas l'association revendiquée.

| **EXEMPLE 9** | |
|---|---|
| Nitrocellulose | 10,84 % |
| Résine toluène sulfonamide formaldéhyde | 9,76 % |
| Acétylcitrate de tributyle | 6,51 % |
| Toluène | 30,97 % |
| Acétate de butyle | 21,685 % |
| Acétate d'éthyle | 9,29 % |
| Alcool isopropylique | 7,74 % |
| Stéaralkonium hectorite | 1,35 % |
| Pigments | 1,00 % |
| Acide citrique | 0,055 % |
| Amino-silicone "KF 865" de la Société Shin-Etsu | 0,3 % |
| Composé organo fluoré "NOFABLE FO-9982" de la Société Nippon Oils | 0,5 % |
| Temps de séchage (30°C-50 % humidité relative) = 14 minutes | |

| **EXEMPLE 10** | |
|---|---|
| Nitrocellulose | 10,84 % |
| Résine toluène sulfonamide formaldéhyde | 9,76 % |
| Acétylcitrate de tributyle | 6,51 % |
| Toluène | 30,97 % |
| Acétate de butyle | 21,685 % |
| Acétate d'éthyle | 9,29 % |
| Alcool isopropylique | 7,74 % |
| Stéaralkonium hectorite | 1,35 % |
| Pigments | 1,00 % |
| Acide citrique | 0,055 % |
| 1-(2'-F-hexyl-éthylthio)-3-(2''-éthylhexyloxy)-2-propanol | 0,5 % |
| Thio-silicone "X22-167B" de la Société Shin-Etsu | 0,3 % |
| Temps de séchage (30°C-50 % humidité relative) = 17 minutes | |

| **EXEMPLE 11** | |
|---|---|
| Nitrocellulose | 10,84 % |
| Résine toluène sulfonamide formaldéhyde | 9,76 % |
| Acétylcitrate de tributyle | 6,51 % |
| Toluène | 30,97 % |
| Acétate de butyle | 21,685 % |
| Acétate d'éthyle | 9,29 % |
| Alcool isopropylique | 7,74 % |
| Stéaralkonium hectorite | 1,35 % |
| Pigments | 1,00 % |
| Acide citrique | 0,055 % |
| 1-(2'-F-hexyl-éthylthio)-3-(2''-éthylhexyloxy)-2-propanol | 0,5 % |
| Thio-silicone "X22-980" de la Société Shin-Etsu | 0,3 % |
| Temps de séchage (30°C-50 % humidité relative) = 16 minutes | |

| **EXEMPLE 12 (comparatif)** | |
|---|---|
| Nitrocellulose | 10,9 % |
| Résine toluène sulfonamide formaldéhyde | 9,8 % |
| Acétylcitrate de tributyle | 6,495 % |
| Toluène | 31,105 % |
| Acétate de butyle | 21,8 % |
| Acétate d'éthyle | 9,395 % |
| Alcool isopropylique | 7,8 % |
| Stéaralkonium hectorite | 1,355 % |
| Pigments | 1,00 % |
| Acide citrique | 0,055 % |
| Thio-silicone "X22-980" de la Société Shin-Etsu | 0,3 % |
| Temps de séchage (30°C-50 % humidité relative) = 22 minutes | |

Cet exemple par rapport à l'exemple 11 montre que l'absence du composé organofluoré hydroxycarboné a pour effet d'augmenter le temps de séchage.

| **EXEMPLE 13** | |
|---|---|
| Nitrocellulose | 10,8 % |
| Résine toluène sulfonamide formaldéhyde | 12,2 % |
| Acétylcitrate de tributyle | 6,52 % |
| Stéaralkonium hextorite | 1,23 % |
| Acétate d'éthyle | 40,38 % |
| Acétate de butyle | 19,3 % |
| Alcool isopropylique | 3,05 % |
| Acide citrique | 0,05 % |
| Pigments | 1,0 % |
| 1-(2'-F-hexyl-éthylthio)-3-(2''-éthylhexyloxy)-2-propanol | 0,3 % |
| Amino-silicone "KF 865" de la Société Shin-Etsu | 0,5 % |
| Temps de séchage (30°C-50 %humidité relative) = 11 minutes | |

| **EXEMPLE 14** | |
|---|---|
| Nitrocellulose | 9,6 % |
| Résine alkyde vendue sous la dénomination de "Beckosol ODE" par la Société Dai-Nippon | 14.63 % |
| Acétylcitrate de tributyle | 7,56 % |
| Stéaralkonium hectorite | 2,43 % |
| Acétate d'éthyle | 31,05 % |
| Acétate de butyle | 24,35 % |
| Alcool isopropylique | 3,72 % |
| Acide citrique | 0,75 % |
| Pigments | 1,0 % |
| 1-(2'-F-hexyl-éthylthio)-3-(2''-éthylhexyloxy)-2-propanol | 0,3 % |
| Amino-silicone "KF 865" de la Société Shin-Etsu | 0,5 % |
| Temps de séchage (30°C-50 % d'humidité relative) = 11,5 minutes | |

## Revendications

1. Vernis à ongles, incolore ou coloré, contenant dans un système solvant pour vernis, une substance filmogène, une résine et un agent plastifiant, caractérisé par le fait que ledit vernis contient en outre, en vue d'améliorer le temps de séchage l'association de (i) 0,05 à 5% en poids d'une amino- ou thio-silicone et de (ii) 0,05 à 5% en poids d'un composé organofluoré hydrocarboné, le poids total de (i) + (ii) devant être inférieur ou égal à 5% du poids total du vernis.

2. Vernis à ongles selon la revendication 1 caractérisé par le fait que l'amino-silicone répond à la formule générale suivante : 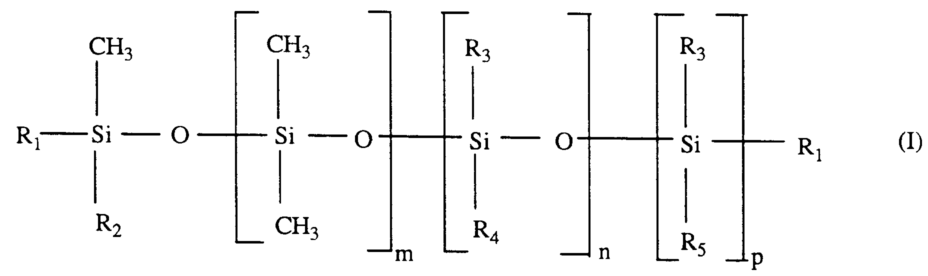 dans laquelle :
R₁ représente CH₃, OCH₃, OCH₂CH₃ ou ―(CH₂)̵_{d}―NH₂
R₂ représente CH₃, OCH₃ ou OCH₂CH₃,
R₃ représente CH₃, OCH₃, OCH₂CH₃ ou OSi(CH₃)₃,
R₄ représente CH₃ ou 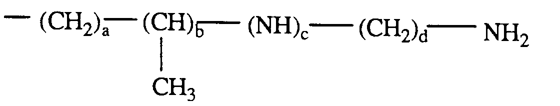
R₅ représente CH₃, OCH₃, OCH₂CH₃ ou 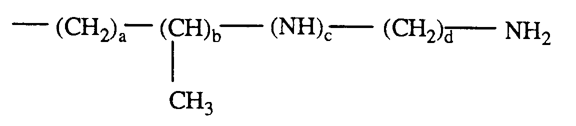
a est 0 à 6
b est 0 à 2
c est 0 ou 1
d est 1 à 6
sous réserve que :
(i) lorsque R₁ représente le radical -(CH₂)_{d}-NH₂, R₄ représente CH₃ et R₅ représente CH₃, OCH₃, ou OCH₂CH₃,
(ii) lorsque R₄ représente CH₃, R₅ représente : 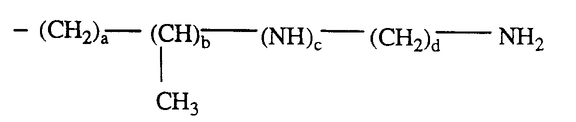 et
(iii) lorsque R₄ représente 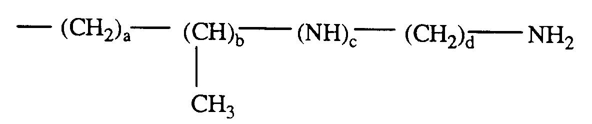
R₅ représente CH₃, OCH₃ ou OCH₂CH₃,
m est 1 à 1000
n est 0 à 50 et
p est 0 à 50
n et p ne pouvant simultanément être O,
le poids moléculaire de la dite amino-silicone étant compris entre 500 et 100.000.

3. Vernis à ongles selon la revendication 1 caractérisé par le fait que la thio-silicone est un polydiorganosiloxane comportant dans la molécule au moins une unité de formule : 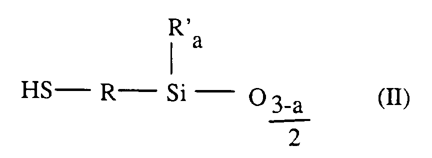 dans laquelle :
R désigne un radical aliphatique divalent ayant de 2 à 26 atomes de carbone, éventuellement interrompu par une fonction ester et pouvant porter des motifs d'oxyde d'éthylène ou d'oxyde de propylène ou leur mélange,
R' désigne un radical monovalent hydrocarbonné ayant 1 à 6 atomes de carbone, alcoxy ayant 1 à 4 atomes de carbone ou triméthylsilyloxy,
a désigne un nombre entier égal à 0, 1 ou 2,
les unités restantes ayant pour formule :
1) 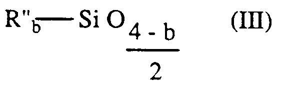 dans laquelle :
R'' représente un groupe alkyle en C₁-C₁₈, phénylalkyle en C₁-C₆ ou phényle,
b est un nombre entier désignant 1, 2 ou 3,
au moins 50% des groupements R' et R'' représentant un groupement méthyle , et
2) éventuellement des unités de formule : 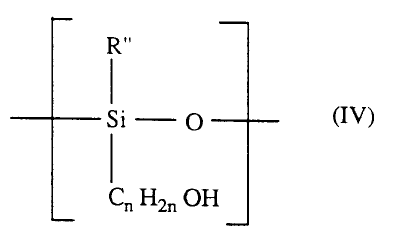 dans laquelle
R'' est tel que défini ci-dessus et n est un nombre entier de 1 à 18.

4. Vernis à ongles selon la revendication 3 caractérisé par le fait que dans l'unité de formule (II), le radical R représente -(CH₂)ₙ- où n est compris entre 3 et 8, ou un groupement de formule :
―CₙH₂ₙO(CₓH₂ₓO)ₚ―COCₘH₂ₘ―
dans laquelle
n représente un nombre entier compris entre 1 et 18, m représente un nombre entier compris entre 1 et 8, x = 2 ou 3 et lorsque x = 3, le radical C₃H₆ est ramifié et p est égal à 0 ou désigne un nombre pouvant aller jusqu'à 40, et
R' représente un radical alkyle en C₁-C₆

5. Vernis à ongles selon l'une quelconque des revendications 1 à 4 caractérisé par le fait que le composé organofluoré hydrocarboné répond à la formule générale suivante :
R₁-(CH₂)ₙ-X-[C₃H₅(OH)]-(Y)ₓ―(CH₂)ₘ―R₂ (VI)
dans laquelle :
le radical divalent C₃H₅(OH) représente l'un au moins des radicaux divalents suivants : 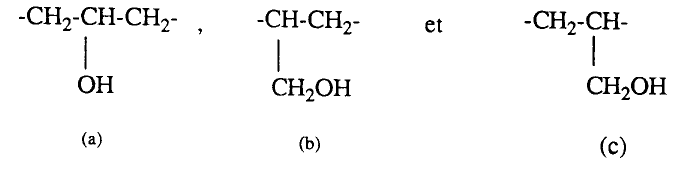
R₁ représente un radical alkyle perfluoré, linéaire ou ramifié, en C₄-C₂₀ ou un mélange de tels radicaux alkyles perfluorés, linéaires ou ramifiés, en C₄-C₂₀,
R₂ représente R₁ ou un radical alkyle, linéaire ou ramifié, en C₁-C₂₂ ou un mélange de tels radicaux alkyles, linéaires ou ramifiés, en C₁-C₂₂ ou un radical aryle en C₆-C₁₀ ou aralkyle en C₇-C₁₅, sous réserve que :
(i) lorsque R₂ est différent de R₁,
X et Y représentent -O- , -S-, 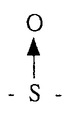 ou 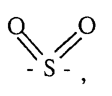 X et Y ne pouvant représenter simultanément 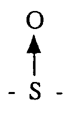 ou 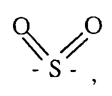 et
(ii) lorsque R₂ représente R₁, X et Y, identiques, représentent -O- ou -S-
m et n, identiques ou différents, sont compris entre 0 et 4 et
x représente O ou 1.

6. Vernis à ongles selon l'une quelconque des revendications précédentes caractérisé par le fait que le composé organofluoré est choisi parmi :
le 1-(2'-F-hexyl-éthylthio)-3-(2''-éthylhexyloxy)-2-propanol,
le 1-(2'-F-octyl-éthylthio)-3-(2''-éthylhexyloxy)-2-propanol,
le 1-(2'-F-octyl-éthylthio)-3-butyloxy-2-propanol,
le 1-(2'-F-octyl-éthylthio)-3-phénoxy-2-propanol,
le 1-(2'-F-hexyl-éthylthio)-3-dodécyloxy-2-propanol,
le 1-(2'-F-hexyl-éthylthio)-2-décanol,
le 1-(2'-F-hexyl-éthylthio)-2-hexanol,
le 1-(2'-F-octyl-éthylthio)-2-hexanol,
le 1-(2'-F-hexyl-éthyloxy)-3-(2''-éthylhexyloxy)-2-propanol,
le 1-(2'-F-hexyl-éthylthio)-3-octylthio-2-propanol,
le 1-(2'-F-octyl-ethylthio)-3-octadécylthio-2-propanol,
le 1,3-bis-(2'-F-hexyl-éthylthio)-2-propanol, et
le 1,3-bis-(2'-F-octyl-éthylthio)-2-propanol.

7. Vernis à ongles selon l'une quelconque des revendications 1 à 4 caractérisé par le fait que le composé organofluoré répond à la formule générale suivante : 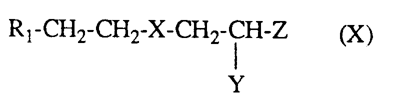 dans laquelle :
R₁ représente un radical alkyle perfluoré, linéaire ou ramifié, en C₄-C₂₀ ou un mélange de tels radicaux alkyles perfluorés, linéaires ou ramifiés, en C₄-C₂₀.
Y est
soit (i) OH et Z représente -CH₃, -CH₂OH, -CH₂OCOCH₃ ou -C₆H₅,
soit (ii) -CH₂OH et Z représente -OCOCH₃, et
X représente -O-, -S-, 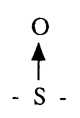 ou

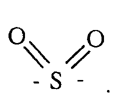

8. Vernis à ongles selon la revendication 7 caractérisé par le fait que le composé organofluoré est choisi parmi :
le 2-(2'-F-hexyl-éthylthio)-1-phényl-éthanol, et
le 2-(2'-F-hexyl-éthylsulfonyl)-1-phényl-éthanol.

9. Vernis à ongles selon l'une quelconque des revendications 1 à 4 caractérisé par le fait que le composé organofluoré répond à la formule générale suivante :
R₁-CH=CH-CH₂-O-[C₃H₅(OH)]―W (XI)
dans laquelle :
le radical divalent C₃H₅(OH) représente l'un au moins des radicaux divalents suivants : 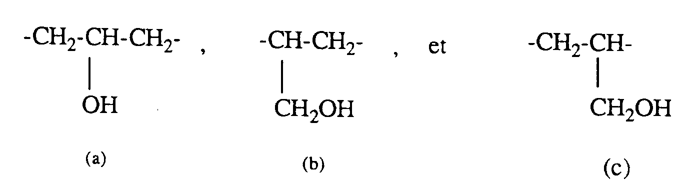
R₁ représente un radical alkyle perfluoré, linéaire ou ramifié, en C₄-C₂₀ ou un mélange de tels radicaux alkyles perfluorés linéaires ou ramifiés, en C₄-C₂₀,
W représente -OR, -SR, -COOR, -O-C₆H₅, o- ou p-O-C₆H₄(CH₃) ou o- ou p-O-C₆H₄(OH), R représentant un radical alkyle, linéaire ou ramifié, en C₁-C₁₈.

10. Vernis à ongles selon la revendication 9 caractérisé par le fait que le composé organofluoré est choisi parmi :
le 1-(3'-F-hexyl-2'-propénoxy)-3-octylthio-2-propanol,
le 1-(3'-F-hexyl-2'-propénoxy)-3-butyloxy-2-propanol, et
le 1-(3'-F-octyl-2'-propénoxy)-3-octadécylthio-2-propanol.

11. Vernis à ongles selon l'une quelconque des revendications 1 à 4 caractérisé par le fait que le composé organofluoré hydrocarboné répond à la formule suivante : 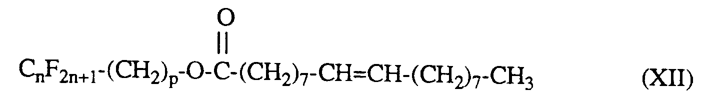 dans laquelle :
n est un nombre entier égal à 6 ou 8 et p est 1 ou 2.

12. Vernis à ongles selon l'une quelconque des revendications précédentes, caractérisé par le fait que le système solvant est présent en une proportion comprise entre 55 et 90 % en poids par rapport au poids total du vernis.

13. Vernis à ongles, selon l'une quelconque des revendications précédentes, caractérisé par le fait que la substance filmogène est présente à une concentration comprise entre 5 et 20 % en poids par rapport au poids total du vernis.

14. Vernis à ongles selon l'une quelconque des revendications précédentes, caractérisé par le fait que la résine est présente en une proportion comprise entre 0,5 et 15 % en poids par rapport au poids total du vernis.

15. Vernis à ongles selon la revendication 14, caractérisé par le fait que la résine aryl sulfonamide formaldéhyde est la résine toluène sulfonamide formaldéhyde.

16. Vernis à ongles selon l'une quelconque des revendications précédentes, caractérisé par le fait que l'agent plastifiant est présent à une concentration comprise entre 2 et 10 % en poids par rapport au poids total du vernis.

17. Vernis à ongles selon l'une quelconque des revendications précédentes, caractérisé par le fait qu'il contient au moins un pigment de nature organique ou inorganique.

18. Vernis à ongles selon la revendication 17, caractérisé par le fait que le pigment est présent à une concentration comprise entre 0,01 et 2 % par rapport au poids total du vernis.

## Claims

1. Coloured or clear nail varnish containing, in a varnish solvent system, a film-forming substance, a resin and a plasticizer, characterized in that the said varnish additionally contains, with a view to improving the drying time, the combination of (i) 0.05 to 5 weight % of an amino- or thiosilicone and (ii) 0.05 to 5 weight % of an organofluorinated hydrocarbon compound, the total weight of (i) + (ii) having to be less than or equal to 5% of the total weight of the varnish.

2. Nail varnish according to Claim 1, characterized in that the aminosilicone corresponds to the following general formula: 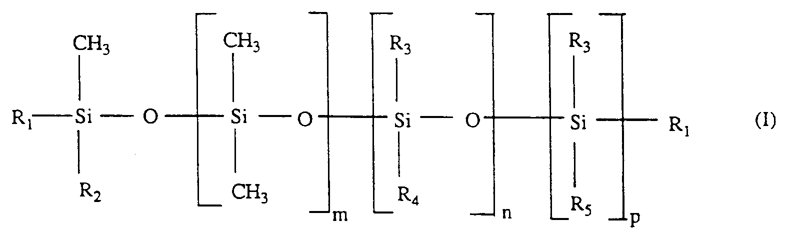 in which:
R₁ represents CH₃, OCH₃, OCH₂CH₃ or -(CH₂)_{d}-NH₂
R₂ represents CH₃, OCH₃ or OCH₂CH₃,
R₃ represents CH₃, OCH₃, OCH₂CH₃ or OSi(CH₃)₃,
R₄ represents CH₃ or 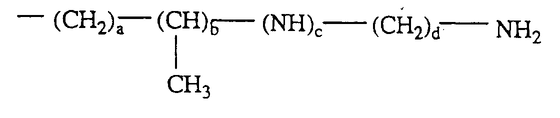
R₅ represents CH₃, OCH₃, OCH₂CH₃ or 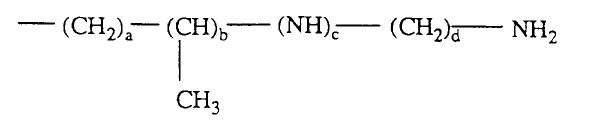
a is 0 to 6
b is 0 to 2
c is 0 or 1
d is 1 to 6
with the proviso that:
(i) when R₁ represents the -(CH₂)_{d}-NH₂ radical, R₄ represents CH₃ and R₅ represents CH₃, OCH₃ or OCH₂CH₃,
(ii) when R₄ represents CH₃, R₅ represents: 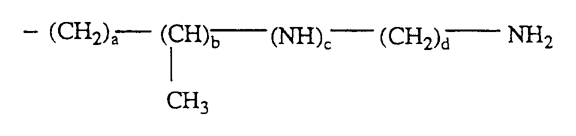 and
(iii) when R₄ represents 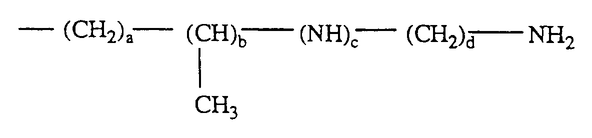
R₅ represents CH₃, OCH₃ or OCH₂CH₃,
m is 1 to 1,000
n is 0 to 50 and
p is 0 to 50
it not being possible for n and p to simultaneously be 0,
the molecular weight of the said aminosilicone being between 500 and 100,000.

3. Nail varnish according to Claim 1, characterized in that the thiosilicone is a polydiorganosiloxane containing, in the molecule, at least one unit of formula: 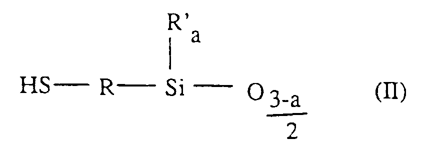 in which:
R denotes a divalent aliphatic radical having from 2 to 26 carbon atoms which is optionally interrupted by an ester functional group and which can carry ethylene oxide or propylene oxide units or their mixture,
R' denotes a monovalent hydrocarbon radical having 1 to 6 carbon atoms, an alkoxy radical having 1 to 4 carbon atoms or a trimethylsilyloxy radical,
a denotes an integer equal to 0, 1 or 2,
the remaining units having the formula:
1) 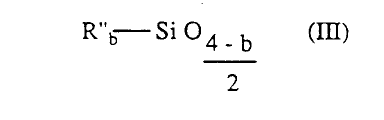 in which:
R'' represents a C₁-C₁₈ alkyl, phenyl(C₁-C₆)alkyl or phenyl group,
b is an integer denoting 1, 2 or 3,
at least 50% of the R' and R'' groups representing a methyl group, and
2) optionally units of formula: 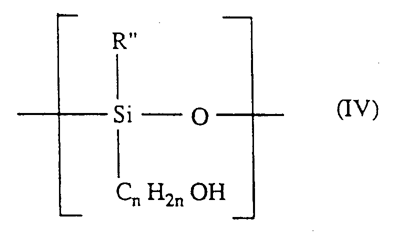 in which
R'' is as defined above and n is an integer from 1 to 18.

4. Nail varnish according to Claim 3, characterized in that in the unit of formula (II), the R radical represents -(CH₂)ₙ-, where n is between 3 and 8, or a group of formula:
-CₙH₂ₙO(CₓH₂ₓO)ₚ-COCₘH₂ₘ-
in which
n represents an integer between 1 and 18, m represents an integer between 1 and 8, x = 2 or 3 and when x = 3, the C₃H₆ radical is branched and p is equal to 0 or denotes a number which can range up to 40, and
R' represents a C₁-C₆ alkyl radical.

5. Nail varnish according to any one of Claims 1 to 4, characterized in that the organofluorinated hydrocarbon compound corresponds to the following general formula:
R₁-(CH₂)ₙ-X-[C₃H₅(OH)]-(Y)ₓ-(CH₂)ₘ-R₂ (VI)
in which:
the divalent radical C₃H₅(OH) represents at least one of the following divalent radicals: 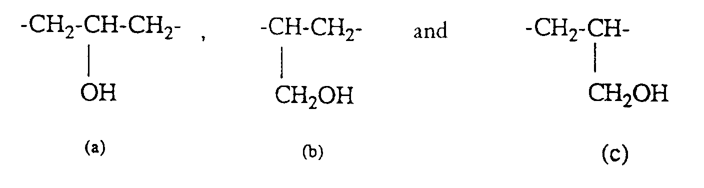
R₁ represents a linear or branched C₄-C₂₀ perfluoroalkyl radical or a mixture of such linear or branched C₄-C₂₀ perfluoroalkyl radicals,
R₂ represents R₁ or a linear or branched C₁-C₂₂ alkyl radical or a mixture of such linear or branched C₁-C₂₂ alkyl radicals or a C₆-C₁₀ aryl or C₇-C₁₅ aralkyl radical, with the proviso that:
(i) when R₂ is different from R₁,
X and Y represent -O-, -S-, 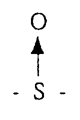 or 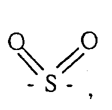 it not being possible for X and Y simultaneously to represent 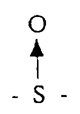 or 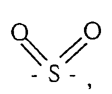 and
(ii) when R₂ represents R₁, X and Y, which are identical, represent -O- or -S-
m and n, which are identical or different, are between 0 and 4 and
x represents 0 or 1.

6. Nail varnish according to any one of the preceding claims, characterized in that the organofluorinated compound is chosen from:
1-(2'-(F-hexyl)ethylthio)-3-(2''-ethylhexyloxy)-2-propanol,
1-(2'-(F-octyl)ethylthio)-3-(2''-ethylhexyloxy)-2-propanol,
1-(2'-(F-octyl)ethylthio)-3-butyloxy-2-propanol,
1-(2'-(F-octyl)ethylthio)-3-phenoxy-2-propanol,
1-(2'-(F-hexyl)ethylthio)-3-dodecyloxy-2-propanol,
1-(2'-(F-hexyl)ethylthio)-2-decanol,
1-(2'-(F-hexyl)ethylthio)-2-hexanol,
1-(2'-(F-octyl)ethylthio)-2-hexanol,
1-(2'-(F-hexyl)ethyloxy)-3-(2''-ethylhexyloxy)-2-propanol,
1-(2'-(F-hexyl)ethylthio)-3-octylthio-2-propanol,
1-(2'-(F-octyl)ethylthio)-3-octadecylthio-2-propanol,
1,3-bis(2'-(F-hexyl)ethylthio)-2-propanol, and
1,3-bis(2'-(F-octyl)ethylthio)-2-propanol.

7. Nail varnish according to any one of Claims 1 to 4, characterized in that the organofluorinated compound corresponds to the following general formula: 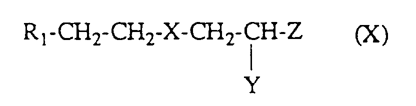 in which:
R₁ represents a linear or branched C₄-C₂₀ perfluoroalkyl radical or a mixture of such linear or branched C₄-C₂₀ perfluoroalkyl radicals,
Y is
either (i) OH and Z represents -CH₃, -CH₂OH, -CH₂OCOCH₃ or -C₆H₅,
or (ii) -CH₂OH and Z represents -OCOCH₃, and
X represents -O-, -S-, 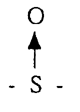 or

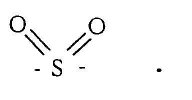

8. Nail varnish according to Claim 7, characterized in that the organofluorinated compound is chosen from:
2-(2'-(F-hexyl)ethylthio)-1-phenylethanol, and
2-(2'-(F-hexyl)ethylsulphonyl)-1-phenylethanol.

9. Nail varnish according to any one of Claims 1 to 4, characterized in that the organofluorinated compound corresponds to the following general formula:
R₁-CH=CH-CH₂-O-[C₃H₅(OH)]-W (XI)
in which:
the divalent radical C₃H₅(OH) represents at least one of the following divalent radicals: 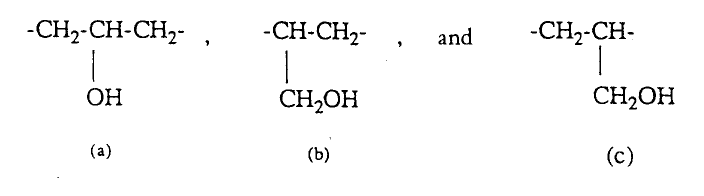
R₁ represents a linear or branched C₄-C₂₀ perfluoroalkyl radical or a mixture of such linear or branched C₄-C₂₀ perfluoroalkyl radicals,
W represents -OR, -SR, -COOR, -O-C₆H₅, o- or p-O-C₆H₄ (CH₃) or o- or p-O-C₆H₄ (OH), R representing a linear or branched C₁-C₁₈ alkyl radical.

10. Nail varnish according to Claim 9, characterized in that the organofluorinated compound is chosen from:
1-(3'-(F-hexyl)-2'-propenoxy)-3-octylthio-2-propanol,
1-(3'-(F-hexyl)-2'-propenoxy)-3-butyloxy-2-propanol, and
1-(3'-(F-octyl)-2'-propenoxy)-3-octadecylthio-2-propanol.

11. Nail varnish according to any one of Claims 1 to 4, characterized in that the organofluorinated hydrocarbon compound corresponds to the following formula: 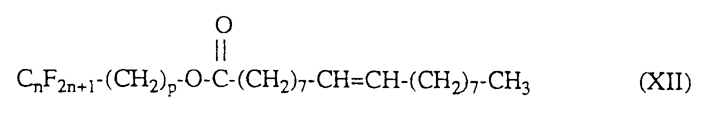 in which:
n is an integer equal to 6 or 8 and p is 1 or 2.

12. Nail varnish according to any one of the preceding claims, characterized in that the solvent system is present in a proportion of between 55 and 90 weight % with respect to the total weight of the varnish.

13. Nail varnish according to any one of the preceding claims, characterized in that the film-forming substance is present at a concentration of between 5 and 20 weight % with respect to the total weight of the varnish.

14. Nail varnish according to any one of the preceding claims, characterized in that the resin is present in a proportion of between 0.5 and 15 weight % with respect to the total weight of the varnish.

15. Nail varnish according to Claim 14, characterized in that the arylsulphonamide-formaldehyde resin is the toluenesulphonamide-formaldehyde resin.

16. Nail varnish according to any one of the preceding claims, characterized in that the plasticizer is present at a concentration of between 2 and 10 weight % with respect to the total weight of the varnish.

17. Nail varnish according to any one of the preceding claims, characterized in that it contains at least one pigment of organic or inorganic nature.

18. Nail varnish according to Claim 17, characterized in that the pigment is present at a concentration of between 0.01 and 2% with respect to the total weight of the varnish.

## Patentansprüche

1. Gegebenenfalls gefärbter Nagellack mit einem Gehalt an einem Lösungsmittelsystem für den Lack, einer filmbildenden Substanz, einem Harz und einem Weichmacher, dadurch gekennzeichnet, daß der Lack zusätzlich noch zur Verbesserung der Trocknungszeit neue Kombination aus
(i) 0,05 bis 5 Gew.-% eines Amino- oder Thiosilikons sowie
(ii) 0,05 bis 5 Gew.-% einer organischen, fluorierten Kohlenwasserstoffverbindung enthält,
wobei das Gesamtgewicht der vorstehenden Komponente (i) + (ii) Weniger oder gleich 5 % in bezug auf das Gesamtgewicht des Lackes ausmacht.

2. Nagellack nach Anspruch 1, dadurch gekennzeichnet, daß das Aminosilikon der folgenden allgemeinen Formel entspricht: 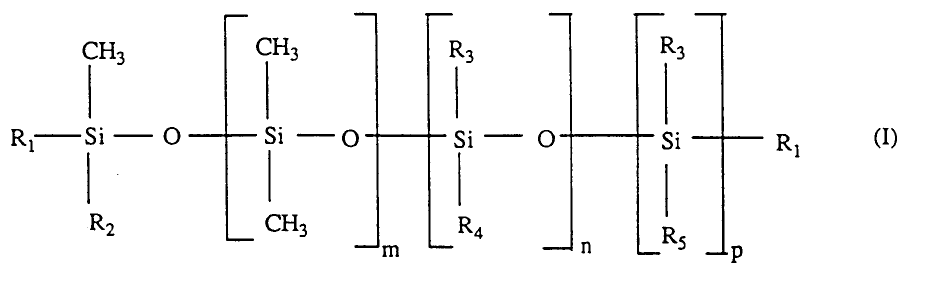 worin
R₁ die Gruppe CH₃, OCH₃, OCH₂CH₃ oder ―(CH₂)_{d}―NH₂,
R₂ die Gruppe CH₃,OHC₃ oder OHC₂CH₃,
R₃ die Gruppe CH₃, OHC₃, OHC₂CH₃ oder OSi(CH₃)₃,
R₄ die Gruppe CH₃ oder 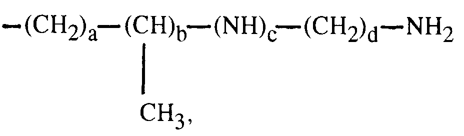 und
R₅ die Gruppe CH₃, OHC₃, OHC₂CH₃ oder 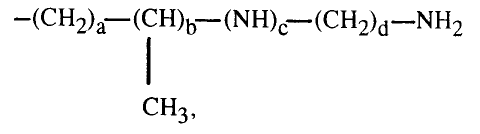 darstellen,
wobei
a die Zahl 0 bis 6
b die Zahl 0 bis 2
c die Zahl 0 oder 1
d die Zahl 1 bis 6
mit der Maßgabe bedeuten,
(i) daß dann, wenn R₁ die Gruppe -(CH₂)_{d}-NH₂ darstellt, R₄ die Gruppe CH₃ sowie R₅ die Gruppe CH₃, OCH₃ oder OCH₂CH₃ bedeuten
(ii) daß dann, wenn R₄ die Gruppe CH₃ bedeutet, R₅ die Gruppe 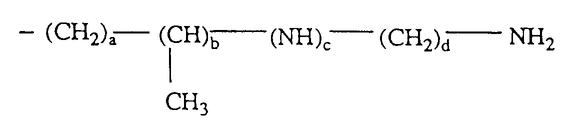 sowie
(iii) dann, wenn R₄ 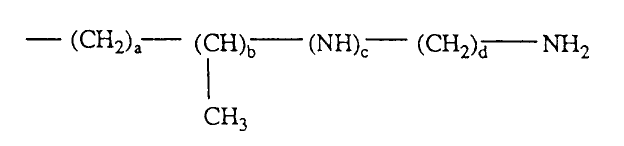 bedeutet, der Rest R₅ die Gruppe CH₃, OCH₃ oder OCH₂CH₃ darstellt, wobei
m die Zahl 1 bis 1000
n die Zahl 0 bis 50 und
p die Zahl 0 bis 50 bedeuten,
n und p nicht gleichzeitig die Zahl 0 sein können und
das Molekulargewicht des genannten Aminosilikons im Bereich zwischen 500 und 100.000 liegt.

3. Nagellack nach Anspruch 1, dadurch gekennzeichnet, daß das Thiosilikon ein Polydiorganosiloxan darstellt, das in dem Molekühl mindestens eine Einheit der folgenden Formel aufweist: 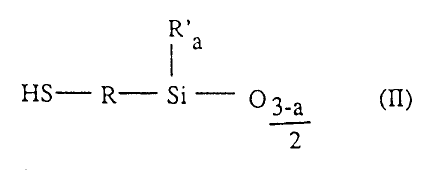 worin
R einen zweiwertigen, aliphatischen Rest mit 2 bis 26 Kohlenstoffatomen bezeichnet, der gegebenenfalls durch eine Esterfunktion unterbrochen sein und Ethylenoxydeinheiten oder Propylenoxydeinheiten bzw. deren Mischung aufweisen kann,
R' einen einwertigen Kohlenwasserstoffrest mit 1 bis 6 Kohlenstoffatomen, Alkoxy mit 1 bis 4 Kohlenstoffatomen oder Trimethylsilyloxy bezeichnet und
a eine ganze Zahl von gleich 0,1 oder 2 bedeutet,
wobei die übrigen Einheiten die folgende Formel aufweisen:
1) 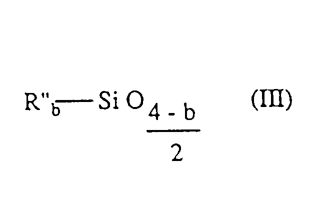 worin
R'' einen C₁-C₁₈-Alkylrest, C₁ bis C₆-Phenylalkylrest oder die Phenylgruppe bedeutet, und
b eine ganze Zahl von 1, 2 oder 3 bedeutet,
wobei mindestens 50 % der Reste R' und R'' eine Methylgruppe darstellen und
2) gegebenenfalls Einheiten den folgenden Formel: 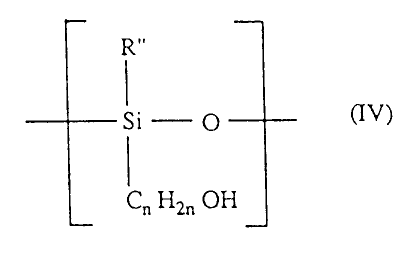 worin
R'' die obenstehende Bedeutung aufweist und n eine ganze Zahl von 1 bis 18 bedeutet.

4. Nagellack nach Anspruch 3, dadurch gekennzeichnet, daß in der Einheit gemäß der Formel (II) der Rest R die Gruppe -(CH₂)ₙ-bedeutet, worin n zwischen 3 und 8 beträgt oder einen Rest der Formel
―CₙH₂ₙO(CₓH₂ₓO)ₚ―COCₘH₂ₘ―
worin
n eine ganze Zahl zwischen 1 und 18 darstellt, m eine ganze Zahl zwischen 1 und 8 darstellt, x = 2 oder 3 und dann, wenn x = 3, die Gruppe C₃H₆ verzweigt ist und p gleich 0 oder eine Zahl bedeutet, die bis zu 40 gehen kann, sowie
R' einen C₁-C₆ Alkylrest bedeutet.

5. Nagellack nach einem der Ansprüche 1 bis 4, dadurch gekennzeichnet, daß die organofluorierte Kohlenwasserstoffverbindung der folgenden allgemeinen Formel entspricht:
R₁-(CH₂)ₙ-X-[C₃H₅(OH)]-(Y)ₓ―(CH₂)ₘ―R₂ (VI)
worin
die zweiwertige Gruppe mit C₃H₅(OH) mindestens eine der folgenden zweiwertigen Gruppen bedeutet: 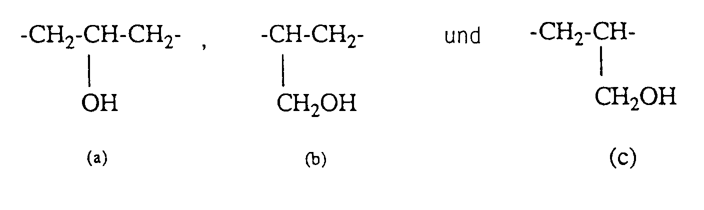
R₁ einen gerad- oder verzweigtkettig perfluorierten C₄-C₂₀-Alkylrest oder ein Gemisch aus diesen perfluorierten gerad- oder verzweigtkettigen C₄-C₂₀ Alkylresten,
R₂ den Rest R₁ oder einen gerad- oder verzweigtkettigen C₁-C₂₂-Alkylrest oder ein Gemisch aus diesem gerad- oder verzweigtkettigen C₁-C₂₂-Alkylresten oder einen C₆-C₁₀-Arylrest bzw. einen C₇-C₁₅-Arylrest mit der Maßgabe bedeutet, daß
(i) dann, wenn R₂ von R₁ verschieden ist,
X und Y die Gruppierungen -O-, -S-, 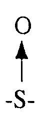 oder 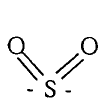 bedeuten, wobei
X und Y nicht gleichzeitig 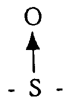 oder 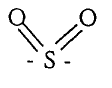 bedeuten können und
(ii) dann, wenn R₂ den Rest R₁ bedeutet,
X und Y als identische Reste -O- oder -S- darstellen und m und n, die gleich oder verschieden sein können, zwischen 0 und 4 bedeuten, sowie
X die Zahl 0 oder 1 darstellt.

6. Nagellack nach einem der vorstehenden Ansprüche, dadruch gekennzeichnet, daß die organofluorierte Verbindung unter den folgenden ausgewählt ist:
1-(2'-F-Hexyl-ethylthio)-3-(2''-ethylhexylody)-2-propanol,
1-(2'-F-Octyl-ethylthio)-3-(2''-ethylhexyloxy)-2-propanol,
1-(2'-F-Octyl-ethylthio)-3-buthyloxy-2-propanol,
1-(2'-F-Octyl-ethylthio)-3-phenoxy-2-propanol,
1-(2'-F-Hexyl-ethylthio)-3-dodecyloxy-2-propanol,
1-(2'-F-Hexyl-ethylthio)-2-decanol,
1-(2'-F-Hexyl-ethylthio)-2-hexanol,
1-(2'-F-Octyl-ethylthio)-2-hexanol,
1-(2'-F-Hexyl-ethyloxy)-3-(2''-ethylhexyloxy)-2-propanol,
1-(2'-F-Hexyl-ethylthio)-3-octylthio-2-propanol,
1-(2'-F-Octyl-ethylthio)-3-octadecylthio-2-propanol,
1,3-Bis-(2'-F-hexyl-ethylthio)-2-propanol, und
1,3-Bis-(2'-F-octylethylthio)-2-propanol.

7. Nagellack nach einem der Ansprüche 1 bis 4, dadurch gekennzeichnet, daß die organofluorierte Verbindung der folgenden allgemeinen Formel entspricht: 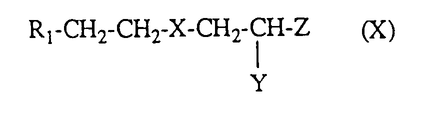 worin
R₁ einen gerad- oder verzweigtkettigen perfluorierten C₄-C₂₀-Alkylrest oder ein Gemisch aus diesen perfluorierten gerad- oder verzweigtkettigen C₄-C₂₀-Alkylresten darstellt,
wobei Y entweder
(i) OH und Z die Gruppe CH₃, CH₂OH, CH₂OCOCH₃ oder C₆H₅ bedeuten,
oder
(ii) die Gruppe CH₂OH und Z die Gruppe OCOCH₃ darstellt, und
X die Gruppierung -O-, -S-, 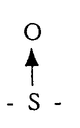 oder 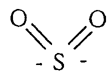 darstellt.

8. Nagellack nach Anspruch 7, dadurch gekennzeichnet, daß die organofluorierte Verbindung unter
2-(2'-F-Hexylethylthio)-1-phenylethanol, und
2-(2'-F-Hexylethylsulfonyl)-1-phenylethanol
ausgewählt ist.

9. Nagellack nach einem der Ansprüche 1 bis 4, dadurch gekennzeichnet, daß die organofluorierte Verbindung der folgenden allgemeinen Formel entspricht:
R₁-CH=CH-CH₂-O-[C₃H₅(OH)]―W (XI)
worin
die zweiwertige Gruppe C₃H₅(OH) mindestens eine der folgenden zweiwertigen Gruppen bedeutet: 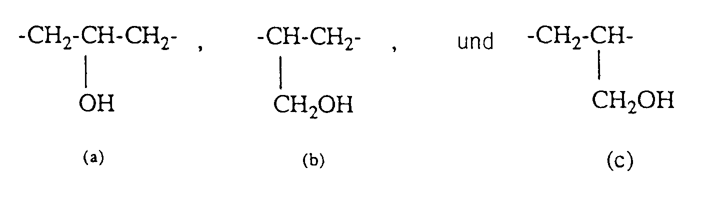 wobei
R₁ einen perfluorierten gerad- oder verzweigtkettigen C₄-C₂₀-Alkylrest oder ein Gemisch aus diesen perfluorierten gerad- oder verzweigtkettigen C₄-C₂₀-Alkylresten bedeutet,
W die Gruppe OR, SR, COOR, O-C₆H₅, o- oder p-OC₆H₄(CH) oder o- oder p-O-C₆H₄(OH) darstellt, wobei R einen gerad- oder verzweigtkettigen C₁-C₁₈-Alkylrest darstellt.

10. Nagellack nach Anspruch 9, dadurch gekennzeichnet, daß die organofluorierte Verbindung aus
1-(3'-F-Hexyl-2'-propenoxy)-3-octylthio-2-propanol,
1-(3'-F-Hexyl-2'-propenoxy)-3-buthyloxy-2-propanol, und
1-(3'-F-Octyl-2'-propenoxy)-3-octadecylthio-2-propanol
ausgewählt ist.

11. Nagellack nach einem der Ansprüche 1 bis 4, dadurch gekennzeichnet, daß die organofluorierte Kohlenwasserstoffverbindung der folgenden Formel entspricht: 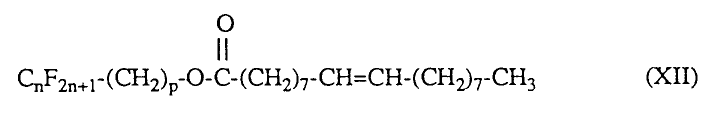 worin
n eine ganze Zahl gleich 6 oder 8 und p die Zahl 1 oder 2 darstellen.

12. Nagellack nach einem der vorstehenden Ansprüche, dadurch gekennzeichnet, daß das Lösungsmittelsystem in einem Mengenverhältnis zwischen 55 und 90 Gew.-% in bezug auf das Gesamtgewicht des Lackes vorhanden ist.

13. Nagellack nach einem der vorstehenden Ansprüche, dadurch gekennzeichnet, daß die filmbildende Substanz in einer Konzentration zwischen 5 und 20 Gew.-% in bezug auf das Gesamtgewicht des Lackes vorhanden ist.

14. Nagellack nach einem der vorstehenden Ansprüche, dadurch gekennzeichnet, daß das Harz in einem Mengenverhältnis zwischen 0,5 und 15 Gew.-% in bezug auf das Gesamtgewicht des Lackes vorhanden ist.

15. Nagellack nach Anspruch 14, dadurch gekennzeichnet, daß das Arylsulfonamidformaldehydharz das Toluolsulfonamidformaldehydharz darstellt.

16. Nagellack nach einem der vorstehenden Ansprüche, dadurch gekennzeichnet, daß der Weichmacher in einer Konzentration zwischen 2 und 10 Gew.-% in bezug auf das Gesamtgewicht des Lackes vorhanden ist.

17. Nagellack nach einem der vorstehenden Ansprüche, dadurch gekennzeichnet, daß er mindestens ein pigmentorganischer oder anorganischer Natur enthält.

18. Nagellack nach Anspruch 17, dadurch gekennzeichnet, daß das Pigment in einer Konzentration zwischen 0,01 und 2 Gew.-% in bezug auf das Gesamtgewicht des Lackes vorhanden ist.
